# EUROPÄISCHE PATENTANMELDUNG

(11) **EP 1 972 293 A2**
(43) Veröffentlichungstag der Anmeldung: **24.09.2008**
(21) Anmeldenummer: 08004484.5
(22) Anmeldetag: 11.03.2008
(51) Int. Cl.: A61B 19/00, A61M 5/20

(54) **Injektionsvorrichtung**

(30) Priorität: 19.03.2007 DE 202007004191 U
(71) Anmelder: Musa, Citak, Dr. med., 30449 Hannover (DE); Hüfner, Tobias, Prof. Dr.med., 30177 Hannover (DE)
(72) Erfinder: Musa, Citak, Dr. med., 30449 Hannover (DE); Hüfner, Tobias, Prof. Dr.med., 30177 Hannover (DE)
(74) Vertreter: Maatz-Jansen, Gero

(57) **Zusammenfassung**

Die Erfindung bezieht sich auf eine Injektionsvorrichtung für die computergestützte Chirurgie mit einer an einem Ende fixierten Hohlnadel (2) und einem mit dieser verbundenen Reservoir (5). Die Injektionsvorrichtung zeichnet sich aus durch einen Referenzstern (3) und eine konische, spitz zulaufende Hülse (1) mit axialer Ausnehmung, in der die Hohlnadel (2) angeordnet ist, wobei die Hülse (1) mit dem Ende in der Injektionsvorrichtung fixiert ist, an dem die Hohlnadel (1) fixiert ist. Die Erfindung bezieht sich auch auf eine mit einer solchen Injektionsvorrichtung verwendbare Parallelführung (20).

## Beschreibung

Die vorliegende Erfindung betrifft eine Injektionsvorrichtung und deren Verwendung für die Injektion pharmazeutischer Zusammensetzungen, in der medizinischtherapeutischen Behandlung mit computergestützter Positionierung, wie beispielsweise für die computergestützte Chirurgie.

Die erfindungsgemäße Injektionsvorrichtung ist insbesondere zur Injektion in der periradikulären Therapie geeignet, weil sie für eine präzise Bestimmung der Positionierung ihrer Hohlnadel geeignet ist. Auf diese Weise enthält die erfindungsgemäße Injektionsvorrichtung eine Vorrichtung zur präzisen Positionierung einer Hohlnadel, ohne dass eine direkte optische Kontrolle der Positionierung des Austrittsendes der Hohlnadel erforderlich wäre.

Die EP 1 543 789 B1 beschreibt ein Instrument für den Einsatz in der computergestützten Chirurgie, dessen Instrumentenschaft einen Referenzstern - Adapter aufweist, der direkt am Instrumentenschaft und um diesen drehbar an einer Adaptionsschnittstelle angeordnet ist. Auf diese Weise soll eine vorherbestimmbare Axialposition des Referenzstern - Adapters bereitgestellt werden.

Die EP 1 733 693 A1 beschreibt die Koordinierung von Geräten und chirurgischen Instrumenten mittels drahtloser Datenübertragung zu einer zentralen Steuerungs- und Empfangseinheit. Zur Ortung der Position von Instrumenten können Referenzsterne eingesetzt werden, die ebenfalls an dem zu behandelnden Patienten fixiert werden können.

Zur periradikulären Therapie, bei der pharmazeutische Zusammensetzungen, beispielsweise Schmerzmittel, zwischen Wirbel injiziert werden, ist es bekannt, die Positionierung der zur Injektion eingesetzten Hohlnadel durch zweidimensionale Röntgendurchleuchtung oder Computertomografie, optional gekoppelt mit einem Bildwandler, zu bestimmen. Für die Bestimmung der Positionierung der Hohlnadel mittels Computertomografie wird der zu behandelnde Patient zunächst computertomografisch abgetastet, wobei in dem erzeugten Bild Punktionsstelle und Einstichwinkel berechnet und auf dem Rücken des Patienten identifiziert werden können. Die Hohlnadel wird anschließend unter computertomografischer Kontrolle vorgeschoben, woraufhin zur Kontrolle der Positionierung der Hohlnadel Kontrastmittel injiziert wird. Nach Erreichen der erwünschten Positionierung der Hohlnadel kann dann ein pharmazeutischer Wirkstoff injiziert werden.

An den bekannten Verfahren zur positionsbestimmten Injektion von pharmazeutischen Zusammensetzungen ist nachteilig, dass eine aufwändige Kontrolle der Positionierung der Hohlnadel durch Röntgen oder Computertomografie erfolgen muss, gegebenenfalls sogar zusätzlich die Injektion eines Kontrastmittels. Dadurch wird die Behandlung technisch und zeitlich aufwändig.

Der vorliegenden Erfindung stellt sich daher die Aufgabe, eine Vorrichtung und ein Verfahren zur periradikulären Therapie bereitzustellen, die die Nachteile des Standes der Technik vermeidet.

Die Erfindung beruht auf der Beobachtung, dass eine Bestimmung der Positionierung der Hohlnadel nach dem Stand der Technik nicht möglich ist, wenn nur die Lage und Ausrichtung des noch sichtbaren Endes der Hohlnadel bestimmbar ist. Denn herkömmliche Hohlnadeln sind elastisch und verändern die lineare Form in Abhängigkeit von der Struktur und Festigkeit des umgebenden Gewebes.

Die Erfindung löst die vorgenannten Aufgaben durch Bereitstellung einer Injektionsvorrichtung, bei der die Hohlnadel durch eine konisch spitz zulaufende Hülse stabilisiert wird, bzw. eine konisch, spitz zulaufenden Hülse bereitgestellt ist, wobei die Hülse mit einem Ende festgelegt ist und die Injektionsvorrichtung einen mit der Hülse verbundenen Referenzstern aufweist. Dabei ist ein mit der Hülse verbundener Referenzstern so mit der Injektionsvorrichtung verbunden, dass die Position der Hülse zu diesem Referenzstern bekannt oder bestimmbar ist und im montierten Zustand unveränderlich ist. So kann der Referenzstern an einem Gehäuse montiert sein, in dem das mit der Hülse bzw. Hohlnadel verbundene Reservoir zur Aufnahme pharmazeutischer Zusammensetzungen angeordnet ist.

Die Kombination der durch die Hülse bereitgestellten Biegefestigkeit der zur Injektion eingesetzten Hohlnadel mit der Bestimmung der Positionierung der Injektionsvorrichtung anhand des Referenzsterns, der mit der Hülse verbunden ist, ermöglicht die Bestimmung der Positionierung der Hülse und damit der Hohlnadel über deren gesamte Baulänge.

Das mit der erfindungsgemäßen Injektionsvorrichtung durchführbare Verfahren beruht auf der Bestimmbarkeit der Positionierung der Hülse und der in dieser oder durch diese bereitgestellten Hohlnadel während der Injektion einer pharmazeutischen Zusammensetzung, vorzugsweise zur periradikulären Therapie. Die Injektionsvorrichtung, die auch eine mit der einseitig fixierten Hohlnadel, bzw. der einseitig fixierten, konisch spitz zulaufenden Hülse verbundene Dosierkammer aufweist, kann mittels ihres Referenzsterns bezüglich ihrer Positionierung detektiert werden. Die relative Positionierung der Injektionsvorrichtung und ihrer Hülse erfolgt durch Korrelation mit der Position des zu behandelnden Patienten. Die Position des zu behandelnden Patienten kann durch Referenzsterne bestimmt werden, die ortsfest mit dem Patienten verbunden sind. Die relative Positionierung eines mit dem Patienten verbundenen Referenzsterns kann durch bekannte Verfahren, beispielsweise durch Röntgen oder Computertomografie, bestimmt oder bestätigt werden, und ist vorzugsweise kontinuierlich erfassbar. Das mit der erfindungsgemäßen Vorrichtung durchführbare Verfahren umfasst daher die Bestimmung der Position der Hülse durch Messung der Position des Referenzsterns, vorzugsweise in Verbindung mit der Messung der Position eines mit dem Patienten verbundenen Referenzsterns, anschließendes Vorschieben der Hülse in ihrer Längsachse in den Patienten unter Bestimmung ihrer Position durch Messung der Position des Referenzsterns der Injektionsvorrichtung.

Die relative Positionierung der Hülse und der mit ihr verbundenen Hohlnadel zur Injektion relativ zu dem Referenzstern der Injektionsvorrichtung ist auf Grund der Abmessungen der Injektionsvorrichtung und der Lage der Hülse in Bezug auf den Referenzstern bestimmbar, bzw. bekannt. Entsprechend ist die Positionierung der Hülse und damit der Hohlnadel aus der Positionierung des Referenzsterns der Injektionsvorrichtung ableitbar.

In bevorzugter Ausführungsform ist die konisch, spitz zulaufende Hülse mit der in ihrer axialen Ausnehmung angeordneten Hohnadel einstückig ausgebildet.

Zur Bestimmung der Positionen von Referenzsternen in Bezug auf ein angelegtes Koordinatensystem, bzw. relativ zueinander, können Referenzsterne unter Zuhilfenahme eines speziellen Kalibrierblocks (ICM, Instrument Calibration Matrix) kalibriert werden. Als Alternative zu dieser sogenannten Vorkalibrierung kann die Positionsbestimmung durch jedes bekannte Erfassungssystem für Referenzsterne erfolgen. Vorzugsweise erfolgt die Positionsbestimmung der Referenzsterne kontinuierlich, beispielsweise durch Positionsbestimmung mittels Messung der von den Positionssternen reflektierten Strahlung, insbesondere Infrarotstrahlung. Entsprechend betrifft die Erfindung auch die Kombination der erfindungsgemäßen Injektionsvorrichtung mit einem System zur Positionsbestimmung von Referenzsternen mit einer Einrichtung zur Abstrahlung von Strahlung und zur Detektion reflektierter Strahlung, vorzugsweise von IR-Strahlung. Weiter bevorzugt weist die Vorrichtung zur Positionsbestimmung der Referenzsterne an der Injektionsvorrichtung und am Patienten eine Ausgabeeinheit zur bildlichen Darstellung der Positionen der Referenzsterne auf, in der auch die aus der Positionierung der Referenzsterne zueinander errechnete Position der Hülse darstellbar ist.

In einer weniger bevorzugten Ausführungsform kann die Hülse die konische, spitz zulaufende Form dadurch aufweisen, dass sich ihr Außendurchmesser abschnittsweise stufenförmig von dem Ende, an dem sie in der erfindungsgemäßen Vorrichtung fixiert ist, verjüngt.

In weiter bevorzugter Ausführungsform ist die mit der Hohlnadel verbundene Dosierkammer, die die zu injizierende pharmazeutische Zusammensetzung enthält, mittels eines Pistolengriffs bedienbar. Ein Pistolengriff, der an der Injektionsvorrichtung zur Bedienung der Dosierkammer angeordnet ist, ermöglicht die Dosierung der zu injizierenden pharmazeutischen Zusammensetzung bei gleichzeitiger Vereinfachung der Handhabung der Vorrichtung, insbesondere zur sicheren manuellen Führung der Injektionsvorrichtung an dem Pistolengriff.

In weiter bevorzugter Ausführungsform weist die erfindungsgemäße Injektionsvorrichtung eine Führungsschiene auf, an der sie in Richtung der Achse der Hülse verschieblich geführt werden kann, während die Positionierung der Schiene in jeder Richtung im Raum eingestellt und fixiert werden kann. Die Schiene ist beispielsweise mittels eines einstellbaren Arms in einer gewünschten Position relativ zu einem Operationstisch fixierbar, beispielsweise am Operationstisch befestigt. Auf diese Weise wird die Bewegung der Hülse der Injektionsvorrichtung in Richtung ihrer Achse auf die Verschiebung entlang der Schiene beschränkt und kann daher bei vorheriger Fixierung der Schiene relativ zu einem Operationstisch, bzw. relativ zu einem Patienten, vorherbestimmt werden.

Die Erfindung wird nun genauer mit Bezug auf die Figuren beschrieben, in denen

Fig. 1 eine schematische Ansicht einer ersten Ausführungsform der erfindungsgemäßen Injektionsvorrichtung zeigt,

Fig. 2 eine schematische Ansicht einer zweiten Ausführungsform mit konischer, spitz zulaufender Hülse zeigt

Fig. 3 eine schematische Ansicht einer weiteren Ausführungsform der erfindungsgemäßen Injektionsvorrichtung mit einer Führung zeigt

Fig. 4a eine schematische Ansicht einer bevorzugten Ausführung der Parallelführung für die erfindungsgemäße Injektionsvorrichtung,

Fig. 4b eine um 90° gedrehte Ansicht von Fig. 4a und

Fig. 4c eine Aufsicht von rechts auf Fig. 4b.

In den Figuren bezeichnen gleiche Bezugsziffern funktionsgleiche Teile.

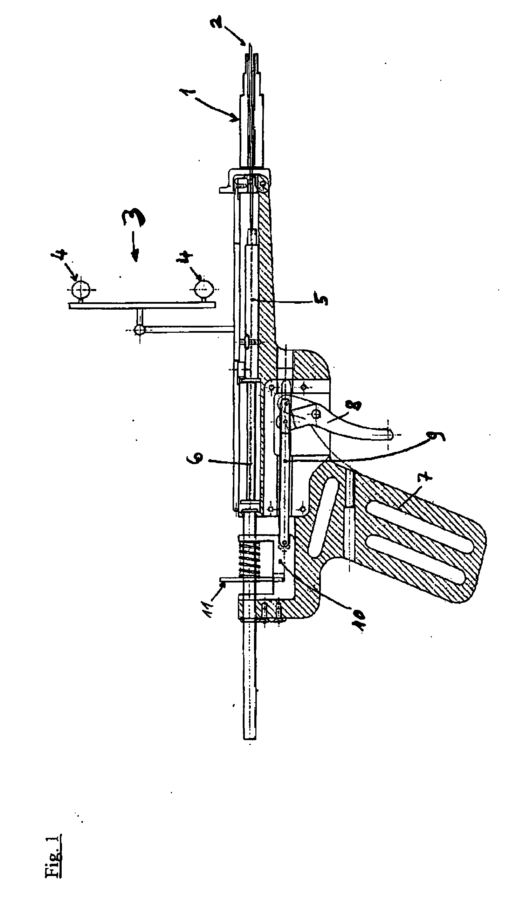

Die Ausführungsform von Fig. 1 zeigt die Hülse 1 und mit einer darin axial angeordneten Hohlnadel 2 an einem Ende der Injektionsvorrichtung. Zur Erfassung der Position der Injektionsvorrichtung weist diese einen Referenzstern 3 auf, der fixierbar ist. Der Referenzstern 3 weist zumindest drei Abschnitte auf, anhand derer seine Positionierung im Raum, z.B. im Verhältnis zu einer Detektionseinheit oder im Verhältnis zu einem anderen Referenzstern, bestimmbar ist. Vorzugsweise sind diese Abschnitte Kugeln. In den Figuren sind zwei Kugeln 4 eines Referenzsterns schematisch dargestellt.

In dieser Ausführungsform ist die Hülse 1 so ausgebildet, dass sie sich abschnittsweise von dem Ende, das festgelegt ist, zu dem Ende, das frei liegt und zum Einstechen eingesetzt werden kann, stufenförmig verjüngt. Weiter dargestellt ist die Ausführungsform, in der die Hülse 1 eine Hohlnadel 2 umgibt. Alternativ ist es auch möglich, dass Hülse 1 und Hohlnadel 2 einstückig geformt sind.

Die Injektionsvorrichtung weist ein mit der Hohlnadel 2 verbundenes Reservoir 5 zur Aufnahme einer pharmazeutischen Zusammensetzung auf, das mittels eines verschieblichen Kolbens 6 durch die Hohlnadel 2 entleert werden kann. Als Alternative zu einem Kolben als Betätigungsvorrichtung für das Entleeren des Reservoirs 5 ist auch eine andere Einrichtung zur Erzeugung eines Überdrucks in dem Reservoir 5 einsetzbar, beispielsweise eine Pumpe.

Zur Betätigung des Kolbens 6 weist die Injektionsvorrichtung vorzugsweise einen Pistolengriff 7 auf, in dem ein Schwenkhebel 8 verschwenkbar angeordnet ist, wobei die Verschwenkung des Schwenkhebels 8 zum Vorschub des Kolbens 6 in das Reservoir 5 führt. Zur Übertragung der vom Schwenkhebel 8 auf den Kolben 6 ausgeübten Kraft ist beispielhaft ein Ende eines Zugstücks 9 mit seinem einen Ende des Schwenkhebels 8 verbunden, und mit seinem anderen Ende mit einem parallel und in Richtung der Hohlnadel 2 verschieblichen Druckstück 10. Das Druckstück 10 ist vorzugsweise mit einer Rasteinrichtung 11 versehen, die ein Verschieben des Druckstücks 10 nur in Richtung auf die Hohlnadel 2 erlaubt, jedoch ein Zurückrutschen von der Hohlnadel 2 weg verhindert.

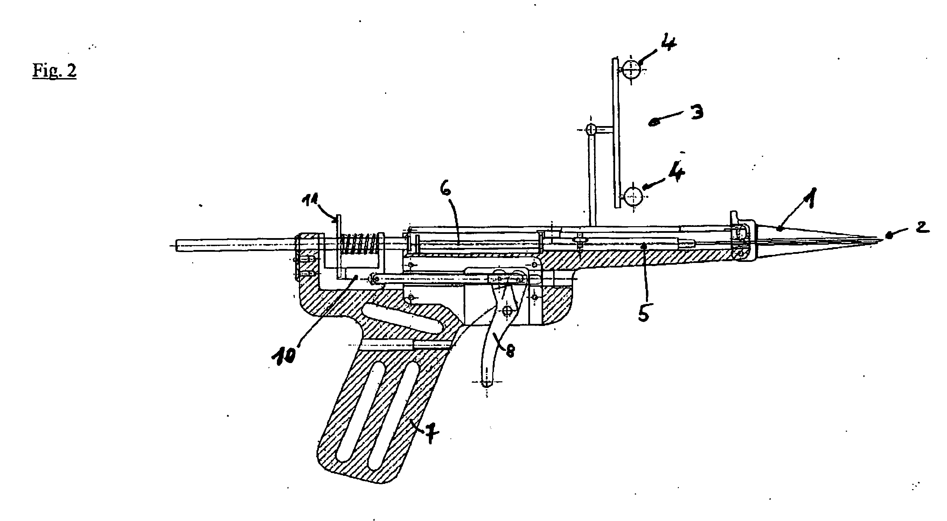

Fig. 2 zeigt eine zweite Ausführungsform der vorliegenden Erfindung, bei der sich die Hülse 1 nicht stufenförmig verjüngt, sondern kontinuierlich und z.B. kegelförmig spitz zuläuft. Bei dieser Ausführungsform ist es auch möglich, dass die Hohlnadel in einem ersten Abschnitt, der an das Ende angrenzt, mit dem die Hülse 1 in der Vorrichtung fixiert ist, einen konstanten Durchmesser aufweist, z.B. in Form eines Rohrs, und einen anschließenden zweiten Abschnitt, der sich bis zur freien Spitze der Hülse 1 erstreckt, wobei nur dieser zweiter Abschnitt kegelförmig spitz zuläuft.

Auch bei dieser zweiten Ausführungsform kann die Hülse 1 einstückig mit der darin angeordneten Hohlnadel 2 sein.

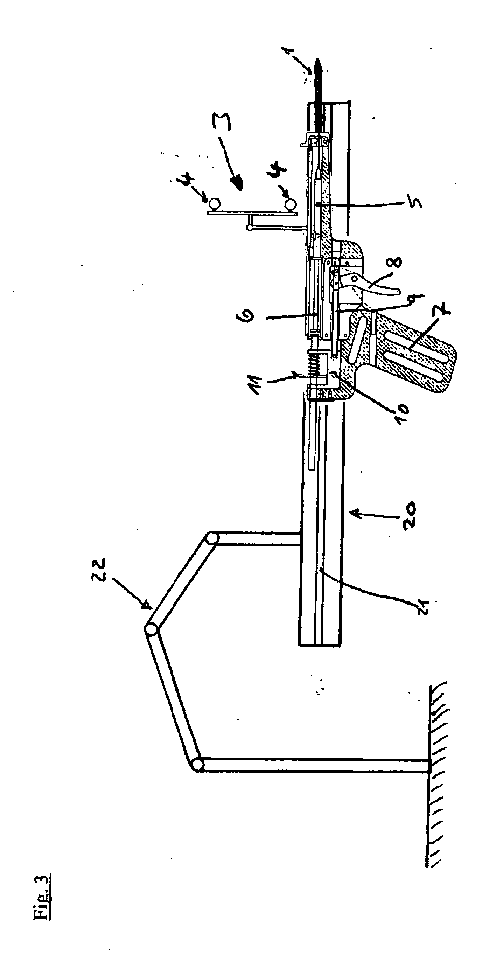

Fig. 3 zeigt schematisch die Anordnung einer erfindungsgemäßen Injektionsvorrichtung auf einer Parallelführung, wobei die Injektionsvorrichtung so auf der Parallelführung 20 angeordnet ist, dass sie nur noch entlang der Achse der Hülse 1 verschieblich ist. Die Parallelführung 20 kann z.B. eine Nut 21 aufweisen, in der dreh- und kippsicher die Injektionsvorrichtung verschieblich ist. Die Parallelführung ist an einem Arm 22 angeordnet, der in jede beliebige Position in Bezug auf eine Unterlage 23 beweglich ist und in dieser Position fixierbar ist.

Die erfindungsgemäße Injektionsvorrichtung ermöglicht anhand des Referenzsterns 3 die Bestimmung der Position der Injektionsvorrichtung, und damit der Anordnung ihrer Hülse 1 mit der darin geführten oder einstückig geformten Hohlnadel 2. Der Pistolengriff 7 ermöglicht eine einfache Handhabung der Injektionsvorrichtung, was für die praktische Anwendung wesentlich ist, da für die Injektion nicht unerhebliche Kräfte überwunden werden müssen.

Die besondere Ausführungsform der Hülse 1 verhindert deren Verbiegung während des Einführens der Hülse 1 in den Körper des Patienten, sodass auch während der Verwendung anhand der Position des Referenzsterns 3 die Position der Hülse 1 mit der Hohlnadel 2 feststellbar ist. In Kombination mit der Bestimmung der Positionierung der gewünschten Einstichstelle und Einstichrichtung, beispielsweise anhand der Positionierung eines zweiten Referenzsterns, der in bekannter Position relativ zur gewünschten Einstichstelle angeordnet und orientiert ist, ermöglicht die erfindungsgemäße Injektionsvorrichtung die präzise und vorherbestimmbare Einführung der Hülse 1 mit der darin angeordneten Hohlnadel 2. Auf diese Weise wird ein unkontrolliertes Eindringen einer Hohlnadel vermieden, wie sie beispielsweise mit unzureichender Festigkeit gegen die Verbiegung aus dem Stand der Technik bekannt ist.

Zur Vermeidung von Positionierungsfehlern der Hülse 1 ist bevorzugt, die erfindungsgemäße Injektionsvorrichtung auf einer Parallelführung 20 anzuordnen, sodass die Hülse 1 ausschließlich in Richtung ihrer Achse verschieblich ist. Die Ausrichtung der Parallelführung 20 führt daher zur Ausrichtung des Referenzsterns 3 der Injektionsvorrichtung, sodass eine vorbestimmte, gewünschte Position und Orientierung relativ zur gewünschten Einstichstelle möglich ist. Die Fixierung der Parallelführung 20 in der gewünschten Position und Orientierung erfolgt durch Arretierung des Arms 22. Der Vorschub der Injektionsvorrichtung entlang der Parallelführung 20kann durch manuelle Betätigung erfolgen, oder durch motorischen Vortrieb, beispielsweise computergesteuert mit einem elektrischen Antrieb Zur Behandlung eines Patienten mit der erfindungsgemäßen Vorrichtung ist es bevorzugt, die Injektionsvorrichtung relativ zu einem zweiten Referenzstern, der mit dem Patienten verbunden ist, so auszurichten, dass der Referenzstern 3 der Injektionsvorrichtung so positioniert und ausgerichtet wird, dass die Hülse 1 die gewünschte Position und Orientierung einnimmt. Ausüben von Druck auf das Reservoir 5 wird die darin befindliche pharmazeutische Zusammensetzung durch die Hohlnadel 2 austreten gelassen. Die Anordnung der Hohlnadel 2 innerhalb der Hülse 1 verhindert eine wesentliche Verbiegung der Hohlnadel 2, sodass eine Abweichung der tatsächlichen Positionierung und Orientierung der Hohlnadel 2 innerhalb des Patienten von der Positionierung und Orientierung im Wesentlichen vermieden wird, die sich aus der Anordnung der Hülse 1 innerhalb der Injektionsvorrichtung ergibt.

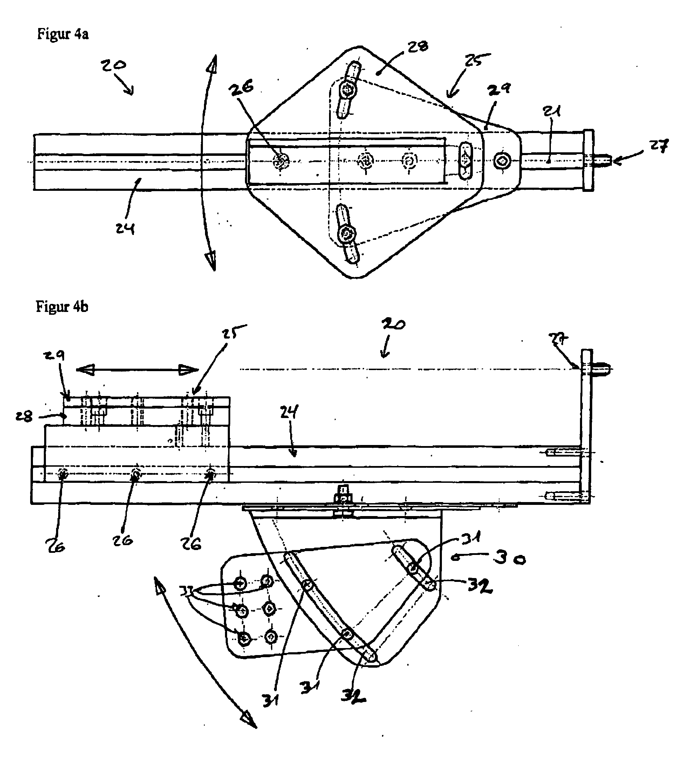

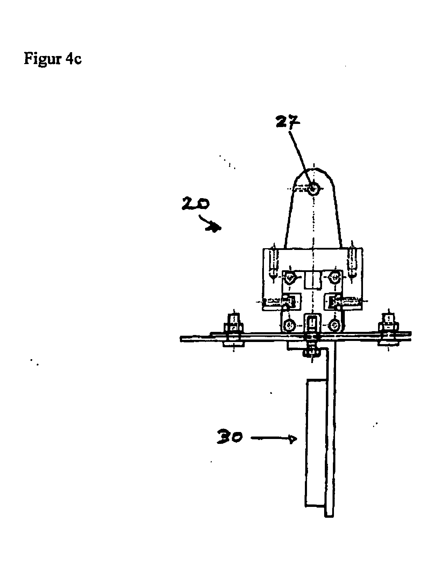

Fig. 4a-c zeigen im Detail eine Parallelführung 20, mit der eine Orientierung und Positionierung der Hülse nach Fixierung der Parallelführung nur in Richtung der Hohlnadel möglich ist. Die Parallelführung weist eine Nut 21 in einer Schiene 24 auf, in der ein Träger 25 mittels Führungselementen 26, z.B. Führungsstiften, verschieblich geführt ist. Die Injektionsvorrichtung, vorzugsweise mit Pistolengriff, kann am Träger 25 montiert werden. In der Folge ist die Hülse (nicht dargestellt) nur parallel zur Nut 21 verschieblich. Bei der Verwendung der Parallelführung für medizinische Zwecke ergibt sich daher der Vorteil, eine Einstichstelle und Einstichrichtung durch Positionierung der Parallelführung einstellen zu können und diese Positionierung durch Fixierung des Arms 22 zu fixieren. Anschließend ist die Hülse nur noch parallel zur Nut 21 verschieblich. Vorzugsweise ist die Hülse parallel zur Nut 21 angeordnet, so dass sie auf der Parallelführung nur in Richtung ihrer Achse angeordnet verschieblich ist.

Zur Führung der Hülse kann die Parallelführung 20 eine Bohrung oder seitlich zugängliche Führungsausnehmung 27 aufweisen.

Der Träger 25 kann zweiteilig ausgeführt sein und eine erste Trägerplatte 28 aufweisen, die mittels Führungselementen 26 in der Nut 21 verschieblich gelagert ist, und eine zweite Trägerplatte 29, die parallel zur ersten Trägerplatte 28 verschwenkbar ist. Die zweite Trägerplatte 29 dient dann zur Aufnahme der Injektionsvorrichtung und kann mit dieser gegen die erste Trägerplatte 28 verschwenkt werden. Auf diese Weise kann die Injektionsvorrichtung, bzw. deren Hülse, in ihrer Position zur Nut 21 der Parallelführung 20 eingestellt werden.

Die Parallelführung 20 weist vorzugsweise eine Schwenkeinrichtung 30 auf, deren Schwenkachse in einem Winkel, vorzugsweise senkrecht, zur Führungsnut 21 angeordnet ist. Diese Schwenkeinrichtung 30 kann zwei parallel angeordnete Elemente aufweisen, die um eine arretierbare Schwenkachse (nicht dargestellt) verschwenkbar sind, und/oder durch Stifte 31 des einen Elements, die in einem oder mehr konzentrischen bogenförmigen Langlöchern 32 des anderen Elements geführt sind. Bohrungen 33 dienen zur Fixierung an einem Arm 22.

Alternativ zur hier dargestellten Nut 21 der Parallelführung 20 kann die Führung des Trägers 25 auch durch andere Funktionselemente realisiert werden, die eine Parallelverschieblichkeit des Trägers 25 entlang einer Schiene 24 ermöglichen.

Zur Bestimmung der Position der Parallelführung 20 in der computergestützten Chirurgie kann die Parallelführung 20 mit einem oder mehreren Referenzsternen versehen sein, beispielsweise an der Schien 24, um die relative Ausrichtung von Injektionsvorrichtung und Schiene bestimmen zur können.

Die hier zur Verwendung mit der Injektionsvorrichtung beschriebene Parallelführung kann auch zur Halterung anderer chirurgischer Instrumente eingesetzt werden, um deren Parallelverschieblichkeit entlang der Nut 21 zu realisieren. Solche anderen chirurgischen Instrumente können z.B. Bohrer und Schraubendreher sein.

### Bezugszeichenliste

1 : Hülse
2 :Hohlnadel
3 :Referenzstern
4 : Kugel
5 :Reservoir
6 :Kolben
7 :Pistolengriff
8 :Schwenkhebel
9 :Zugstück
10 :Druckstück
11 :Rasteinrichtung
20 :Parallelführung
21 : Nut
22 :Arm
23 : Unterlage
24 : Schiene
25 : Träger
26 : Führungselement
27 : Führungsausnehmung
28 : erste Trägerplatte
29 : zweite Trägerplatte
30 : Schwenkeinrichtung
31 : Stift
32 : Langloch
33 : Bohrung

## Patentansprüche

1. Injektionsvorrichtung für die computergestützte Chirurgie mit einer an einem Ende fixierten Hohlnadel (2) und einem mit dieser verbundenen Reservoir (5),
**gekennzeichnet durch** einen Referenzstern (3) und eine konische, spitz zulaufende Hülse (1) mit axialer Ausnehmung, in der die Hohlnadel (2) angeordnet ist, wobei die Hülse (1) mit dem Ende in der Injektionsvorrichtung fixiert ist, an dem die Hohlnadel (1) fixiert ist.

2. Injektionsvorrichtung nach Anspruch 1, **dadurch gekennzeichnet, dass** sich die Hülse (1) abschnittsweise stufenförmig verjüngt.

3. Injektionsvorrichtung nach einem der vorangehenden Ansprüche, **dadurch gekennzeichnet, dass** Hülse (1) und Hohlnadel (2) einstückig sind.

4. Injektionsvorrichtung nach einem der vorangehenden Ansprüche, **dadurch gekennzeichnet, dass** sie einen Pistolengriff (7) mit einem Schwenkhebel (8) aufweist, dessen eines Ende mit einem Druckstück (10) verbunden ist, der gegen einen Kolben (6) verschieblich ist, der in das Reservoir (5) verschieblich ist.

5. Injektionsvorrichtung nach Anspruch 4, **dadurch gekennzeichnet, dass** das Druckstück (10) mit einer Rasteinrichtung (11) versehen ist, die ein Verschieben des Druckstücks (10) nur in Richtung auf das Reservoir (5) zulässt.

6. Injektionsvorrichtung nach einem der vorangehenden Ansprüche, **dadurch gekennzeichnet, dass** die Injektionsvorrichtung an einer Parallelführung (20), die mittels eines Arms (22) relativ zu einer Unterlage (23) positionierbar und fixierbar ist, nur parallel zur Achse der Hülse (1) verschieblich angeordnet ist.

7. Parallelführung (20) zur Verwendung mit einer Injektionsvorrichtung nach einem der vorangehenden Ansprüche, **gekennzeichnet durch** eine Schiene (24) und einen parallel zur Längsachse der Schiene (24) verschieblich angeordneten Träger (25) zur Aufnahme eines chirurgischen Instruments.

8. Parallelführung (20) nach Anspruch 7, **gekennzeichnet durch** eine Führungsausnehmung (27), die parallel zur Längsachse der Schiene (24) angeordnet ist.
